# EUROPEAN PATENT SPECIFICATION

(11) **EP 3 463 039 B1**
(45) Date of publication and mention of the grant of the patent: **22.04.2026**
(21) Application number: 17810955.9
(22) Date of filing: 07.06.2017
(51) Int. Cl.: A61B 1/00, A61B 1/015, A61B 1/04, A61M 1/00, B29C 45/00, B29C 45/40, B29L 31/00

(54) **SUCTION VALVE**
SAUGVENTIL
SOUPAPE D'ASPIRATION

(30) Priority: 07.06.2016 US 201662346847 P
(43) Date of publication of application: 10.04.2019
(73) Proprietor: United States Endoscopy Group, Inc., Mentor, OH 44060 (US)
(72) Inventor: STILL, Rapheal, Richmond Heights Ohio 44143 (US); MANN, Gary, Painesville Ohio 44077 (US); KAYE, Christopher, Eastlake Ohio 44095 (US)
(74) Representative: Petraz, Gilberto Luigi
(86) International application number: PCT/US2017/036398
(87) International publication number: WO 2017/214300

(56) References cited:
- EP-A1- 2 904 960
- CN-A- 1 133 218
- JP-A- H01 192 518
- JP-B2- 3 660 749
- US-A1- 2003 181 787
- US-A1- 2013 302 848
- US-A1- 2013 338 442
- US-A1- 2013 338 442
- US-B2- 9 585 545

## Description

### BACKGROUND

The present disclosure relates to endoscopic systems, more particularly, suction valves for endoscopes.

Suction valves are used to control the suction function of an endoscope during endoscopic procedures. For example, a suction valve for an endoscope may be inserted into a suction cylinder of the endoscope to provide suction to the endoscope. When the suction valve is in a normal position, suction to the distal tip of the endoscope is blocked by the valve. When suction is desired, an operator engages the suction valve (e.g. by depressing the valve) to open the suction channel to create negative pressure that draws air or fluid into the opening of the instrument channel of the endoscope. When the operator releases the suction valve, the valve returns to its normal position blocking air flow and stops the suctioning. Document US-A-2003/181787 describes a suction valve for endoscope use.

Document EP 2 904 960 A1 discloses a suction conduit switching apparatus for an endoscope comprising a piston having three notch passages formed at 90 degree intervals on the piston main body, wherein the notch passages are formed by cutting the outer peripheral surface of the piston into a planar shape. Together with a circumferential groove which is disposed below the three notch passages and above the bottom of the stem, a communication passage is established.

### SUMMARY

The invention is defined by independent claim 1.

In some embodiments, a suction valve comprises the suction valve stem, a spring, a spring cup, and a boot, wherein the bottom end of the stem extends through the spring, the spring cup, and the boot.

In some embodiments, an endoscopic valve kit comprises a suction valve, an air/water valve, and a biopsy valve.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### BRIEF DESCRIPTION OF THE DRAWINGS

Features and advantages of the general inventive concepts will become apparent from the following detailed description made with reference to the accompanying drawings.
Figure 1A is a perspective view of an embodiment of a suction valve of the present subject matter;
Figure 1B is a cross sectional view of the suction valve in Figure 1A;
Figure 2A is a perspective view of an embodiment of a stem of the present subject matter;
Figure 2B is a side view of the stem in Figure 2A;
Figure 2C is a front view of the stem in Figure 2A;
Figure 2D is a front cross sectional view of the stem in Figure 2A;
Figure 3A is a cross sectional view at the distal end of an embodiment of a stem body of the present subject matter;
Figure 3B is a cross sectional view at the distal end of another embodiment of a stem body of the present subject matter;
Figure 3C is a cross sectional view at the distal end of a third embodiment of a stem body of the present subject matter;
Figure 3D is a cross sectional view at the distal end of a fourth embodiment of a stem body of the present subject matter;
Figure 3E is a cross sectional view at the distal end of a fifth embodiment of a stem body of the present subject matter;
Figure 4 is a side view of the stem of the present subject matter;
Figure 5 is a front view of the stem of the present subject matter;
Figure 6 is a perspective view of the stem of the present subject matter; and
Figure 7 shows a stem made by a molding method embodiment of the present subject matter.

### DETAILED DESCRIPTION

This Detailed Description merely describes exemplary embodiments in accordance with the general inventive concepts and is not intended to limit the scope of the invention or the claims in any way. Indeed, the invention as described by the claims is broader than and unlimited by the exemplary embodiment set forth herein, and the terms used in the claims have their full ordinary meaning.

The general inventive concepts will now be described with occasional reference to the exemplary embodiments of the invention. This general inventive concept may, however, be embodied in different forms and should not be construed as limited to the embodiments set forth herein. Rather, these embodiments are provided so that this disclosure will be thorough and complete, and will fully convey the scope of the general inventive concepts to those skilled in the art.

Unless otherwise defined, all technical and scientific terms used herein have the same meaning as commonly understood by one of ordinary skill in the art encompassing the general inventive concepts. The terminology set forth in this detailed description is for describing particular embodiments only and is not intended to be limiting of the general inventive concepts. As used in this detailed description and the appended claims, the singular forms "a," "an," and "the" are intended to include the plural forms as well, unless the context clearly indicates otherwise.

Unless otherwise indicated, all numbers, such as for example, numbers expressing quantities of ingredients, percentages or proportions of materials, reaction conditions, and other numerical values used in the specification and claims, are to be understood as being modified in all instances by the term "about." Accordingly, unless otherwise indicated, the numerical properties set forth in the specification and claims are approximations that may vary depending upon the suitable properties sought to be obtained in the embodiments of the invention. Notwithstanding that the numerical ranges and parameters setting forth the broad scope of the general inventive concepts are approximations, the numerical values set forth in the specific examples are reported as precisely as possible. Any numerical values, however, inherently contain certain errors necessarily resulting from error found in their respective measurements.

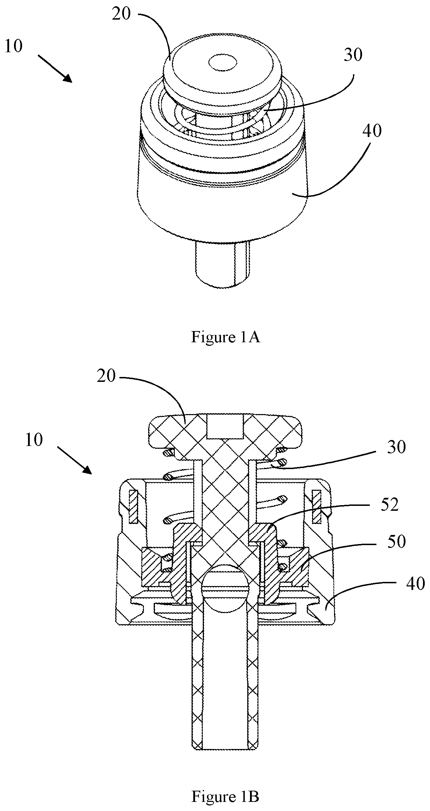

Aspects of the present disclosure relate to suction valves with improved fitment to endoscopes. The present invention also eliminates problems with leaks and leakage seen in other valves. Referring to Figures 1A-1B, an exemplary suction valve 10 comprises a stem 20, a spring 30, a boot 40, and a spring cup 50. The stem 20 extends through the spring 30 and the spring cup 50. In some embodiments, the boot 40 is placed over the spring cup 50. In some other embodiments, the boot 40 is over-molded over the spring cup 50, and therefore the boot 40 and the spring cup 50 are one-piece. The boot 40 comprises a sealing ledge that seals off the suction port in the endoscope. In an additional embodiment it is possible to combine parts 50, 52, and 40 all into a single component (material).

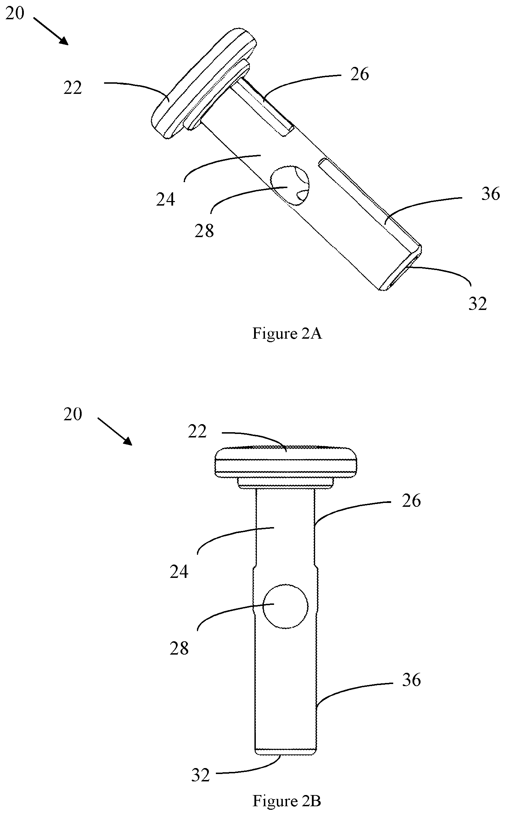

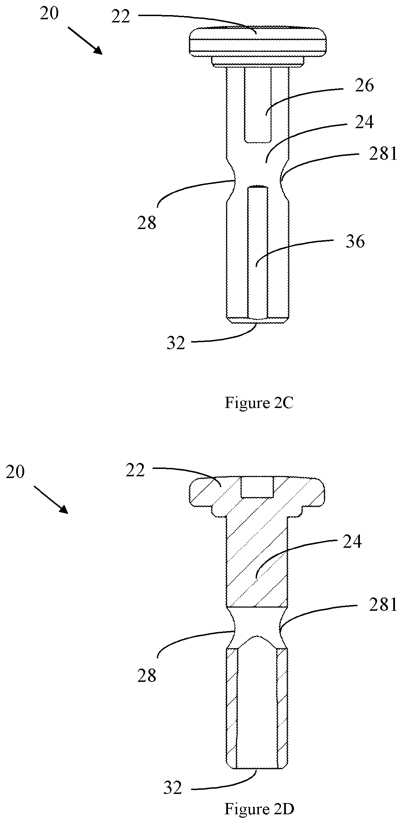

Referring to Figures 1B, 2A-2D, the stem 20 comprises a top part 22 and a stem body 24. In some embodiments, the top part 22 of the stem 20 can also be referred to as a button head or button cap 22. The spring cup 50 comprises tabs 52. The stem 20 further comprises recessed slots 26. The tabs 52 reside in the recessed slots 26 of the stem 20. The recessed slots 26 allow the restricted movement of the stem 20 up and down on the spring cup 50. The spring 30 maintains the stem 20 in its upper position, but the recessed slots 26 and the tabs 52 prevent the stem 20 from being separated from the spring cup 50. When the suction valve 10 is actuated, the spring 30 is compressed and the stem 20 moves further down into the spring cup 50. The recessed slots 26 limit how far down the stem 20 may travel because the tabs 52 will eventually come in contact with the top part of the stem 20.

The stem 20 comprises the button head 22 and the stem body 24. The stem body comprises a side opening 28 and a bottom opening 32. In some embodiments, the stem body comprises a first side opening 28 at one side and a second side opening 281 at the opposite side. Fluid may pass horizontally through one of the first and second side openings 28, 281 and vertically though the bottom opening 32. The side openings 28, 281, and the bottom opening 32 may allow fluid to pass through the instrument channel of an endoscope when the suction valve 10 is actuated.

In some embodiments, the bottom opening 32 is disposed along a longitudinal axis of the stem body 24. The side openings are disposed transverse to the bottom opening 32. In some embodiments, one of the first and second side openings 28, 281 and the bottom opening 32 are perpendicular to each other.

The stem body 24 comprises recessed slots 26. In some embodiments, the stem body 24 comprises two recessed slots 26. In some embodiments, the recessed slots 26 are disposed at the top portion of the stem body 24. In some embodiments, the recessed slots 26 are disposed on the top portion of the opposite surfaces of the top portion of the stem body 24. In some embodiments, the recessed slots 26 are perpendicular to both the first and second side openings 28, 281. The recessed slot 26 orients the first or second side openings 28, 281 in the correct direction when the suction valve 10 is connected to the endoscope.

In some embodiments, the stem 20 functions as a tool the user (clinician) actuates to control vacuum (suction) to the patient. The stem 20 is depressed to allow vacuum suction through an endoscope and is released to stop vacuum suction.

In some embodiments, the interface between the endoscope and the stem 20 is important because the suction valve 10 must actuate smoothly while creating a sealing surface that does not allow suction to be lost during use. If there is a poor interface between the stem and endoscope, a loss of air within a patient will occur due to vacuum suction leaking around the stem. The fitment also needs to generate a hydraulic seal to mitigate fluids suctioned up an endoscope from going back down into a patient. In some embodiments of stem 20, the stem diameter may be precisely controlled to assure an air tight or nearly air tight seal within the suction cylinder/port of an endoscope.

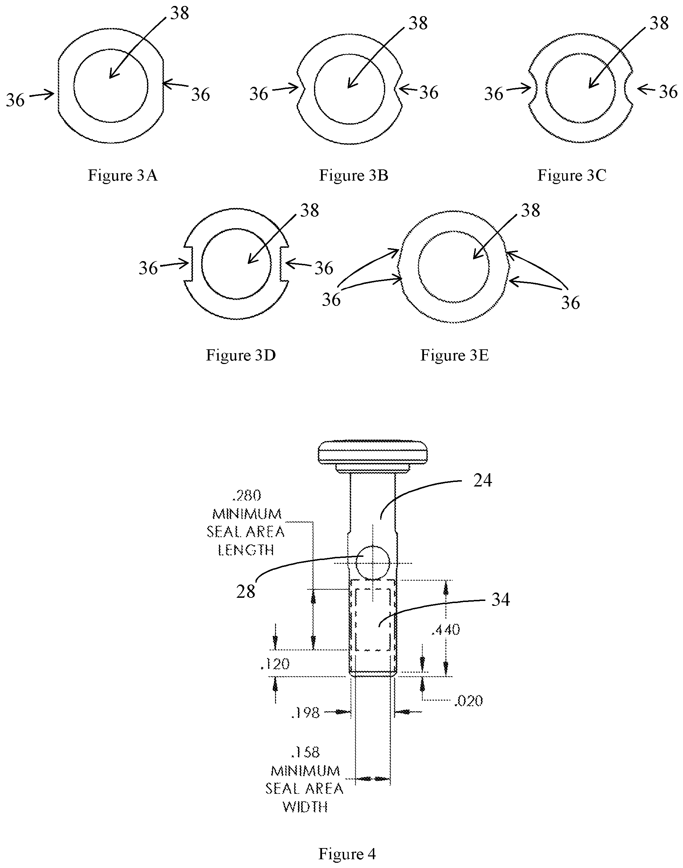

Referring to Figures 4-6, in some embodiments, the stem body 24 comprises one seal area 34. In some embodiments, the stem body 24 comprises two seal areas 34. In some embodiments, the seal areas 34 are disposed on the bottom portion of the stem body 24 and below the side openings 28, 281. In some embodiments, the seal areas 34 are disposed on the opposite surfaces of the stem body 24.

In some embodiments, the seal area 34 may be disposed as high as the first or second side openings 28, 281. In some embodiments, the seal area 34 may be disposed as low as the bottom of the stem body 24. In some embodiments, the seal area 34 may be disposed as narrow as about the diameter of the first or second side openings 28, 281. In some embodiments, the seal area 34 may be disposed as wide as about a half of the perimeter of the stem body 24. In some embodiments, the minimum length of the seal area 34 is about 0.280 inches. In some embodiments, the maximum length of the seal area 34 is about 0.440 inches. In some embodiments, the minimum width of the seal area 34 is about 0.158 inches. In some embodiments, the maximum width of the seal area 34 is about 0.198 inches.

In some embodiments, the seal area 34 or the two seal areas 34 are ribbed and/or textured. However, the seal areas still are able to assure an air tight seal. The ribbed seal areas 34 may provide an easier movement of the stem 20.

In some prior art, however, the substantial air tight seal between the suction cylinder/port of an endoscope and the stem sometimes causes a hydraulic lock when the stem is depressed. Such hydraulic lock may prevent the stem from be releasing back to the upper position.

Referring to Figures 2A, 2C, 5 and 6, in some embodiments, the stem body 24 further comprises flats 36. According to the invention, the stem body 24 comprises two flats which are disposed on the opposite surface of the stem body 24. In some embodiments, the flats 36 are disposed below the recessed slots 26 and on the bottom portion of the stem body 24. According to the invention, the flats 36 extend to the bottom end of the stem body 24. In some embodiments, the flats 36 at least extend to the bottom level of the first or second openings 28, 281. In some embodiments, the flats 36 extend to the middle of the first or second openings 28, 281. In some embodiments, the flats 36 and the recessed slots 26 are separated and therefore prevent fluid communications from each other. In some embodiments, the flats 36 and the first and second side openings 28, 281 are separated and therefore prevent fluid communications from each other.

In some embodiments, the maximum length of the flat 36 is about 0.625 inches. In some embodiments, the minimum length of the flat 36 is about 0.450 inches. In some embodiments, the maximum width of the flat 36 is about 0.103 inches. In some embodiments, the minimum width of the flat 36 is about 0.020 inch.

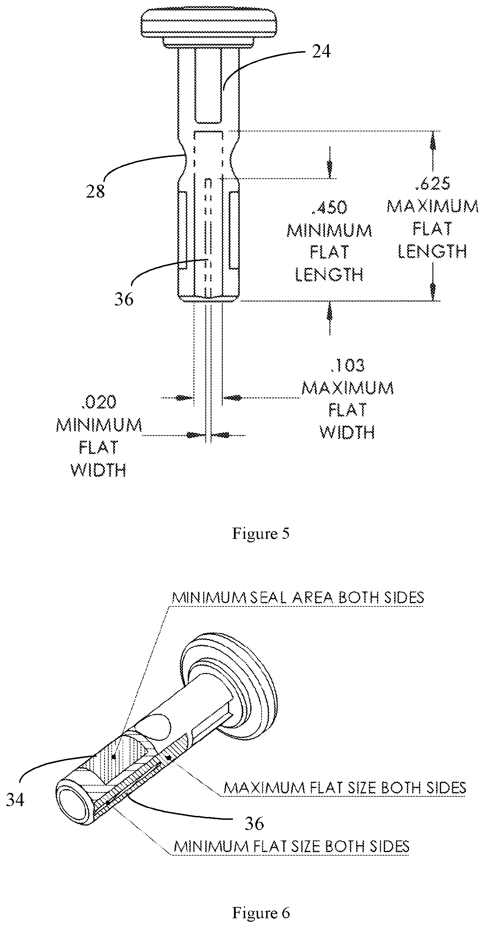

Referring to Figure 6, in some embodiments, the sealing area 34 and the flat 36 do not overlap. In some embodiments, the sealing area 34 and the flat 36 are adjacent to each other.

Referring to Figure 3A, in some embodiments, the cross sectional view of the flat 36 is a plane shape. Referring to Figure 3B, in some embodiments, the cross sectional view of the flat 36 is a concave shape. Referring to Figure 3C, in some embodiments, the cross sectional view of the flat 36 is a pit shape. Referring to Figure 3D, in some embodiments, the cross sectional view of the flat 36 is a slot shape. Referring to Figure 3E, in some embodiments, the cross sectional view of the flat 36 is a four-flat shape. Referring to Figures 3A-3E, in some embodiments, the flat 36 and the internal channel 38 of the stem body are separated and therefore prevent fluid communications from each other. In some embodiments, the flat 36 helps to reduce noise while vacuum suction is in use.

The stem 20 may be formed from a suitable material or combination of material(s), such as plastic, polymeric material(s), metal, or the like. In some embodiments, the button head 22 of the stem 20 is made of plastic or polymeric materials, while the rest part of the stem 20 is made of metal materials. In some other embodiments, the button head 22 is made of plastic or polymeric materials, and the recessed slot part of the stem 20 is made of plastic or polymeric materials; while the rest part of the stem 20 (including of the first and second openings and the flat 28) is made of metal materials. In some other embodiments, the stem 20 is made of metal materials.

In some embodiments, the stem 20 is formed from ABS. Comparing to polycarbonate material often used in prior art, ABS allows the stem 20 to be more compliant in tight tolerance sealing applications.

While the suction valve 10 shown is suitable for use with Olympus^{®} endoscopes, other embodiments of suction valves may be suitable for use with other types of endoscopes, such as Pentax^{®}, Fujinon^{®}, or the like. As such, the embodiments discussed herein may be modified to accommodate other types and/or brands of endoscopes. The suction valve 10 shown here may be used as disposable suction valve. In some other embodiments, the suction valve 10 may be used as reusable/reprocessable suction valve.

In various embodiments, an endoscope valve kit is provided that may include additional parts along with the suction valve combined together to be used with the suction valve. The kit may further include an air/water valve. The endoscopic valve kit may further include a biopsy valve.

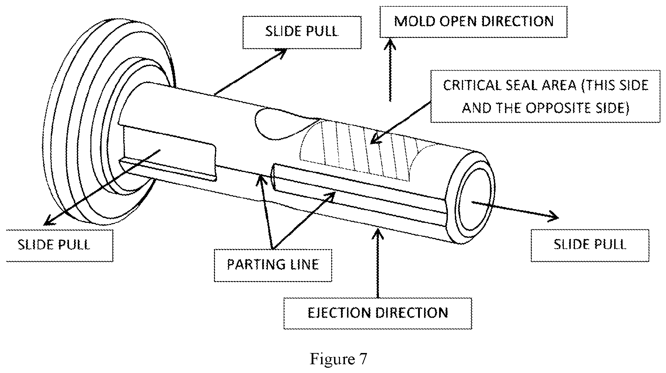

Referring to Figures 7A-7B, in some prior art molding method, the stem has no parting line on the sealing surface. The only parting line is in the non-critical area between the button head and the side openings. The parting line is the witness line of slide face shut offs that pull the retaining grooves. Directional drag marks near the side openings and at the bottom ends of the stem suggest that the stem was pushed out of a mold cavity going from the bottom of the stem to the top of the stem.

Continuing to address the prior art, the through side opening is likely pulled using a mechanical slide or a hydraulic core pull mechanism. This pin is "through the cavity block," which means it operates through an opening in the cavity block below the parting line of the mold. This action can leave burrs on the opening in the cavity block that result in the drag marks seen in the center picture. These drag marks are essentially grooves in the surface of the plastic that prevent the stem from having a smooth sealing surface.

Referring to Figure 8, some embodiments of the present subject matter disclose molding approach to tooling the stem. In specific, the method comprises to place the mold parting line on the non-sealing surfaces such as the flats which are located 90 degrees from the critical sealing areas. In some embodiments, the mold parting line is placed along the length of a non-sealing area. This leaves the critical sealing areas completely free of the tooling marks and leaves no drag marks from ejection from the mold. It allows the sealing areas to stay uniform and smooth and be completely parallel (no draft or taper) to the mating surface of the endoscope. In some embodiments, the cross opening core pins now pull in the same direction as the mold opening direction, leaving no burrs around the side openings that can leave artifacts in the sealing areas that can promote leaks. In some embodiments, the axial opening core pin is pulled in a direction that is perpendicular to the mold opening direction. In some embodiments, the recessed slot cores are pulled in a direction that is perpendicular to the mold opening direction. In some embodiments, the button head core pin is pulled in a direction that is perpendicular to the mold opening direction. In some embodiments, a seal area is formed in opposing halves of a mold cavity. In some embodiments, a sealing area has no draft angle along the length of the sealing area. In some embodiments, the part is ejected from the mold in the same direction as the mold opening direction. In some embodiments, an ejector pin mark near or on the sealing area is recessed.

While various inventive aspects, concepts and features of the general inventive concepts are described and illustrated herein in the context of various exemplary embodiments, these various aspects, concepts and features may be used in many alternative embodiments, either individually or in various combinations and sub-combinations thereof. Unless expressly excluded herein all such combinations and sub-combinations are intended to be within the scope of the general inventive concepts. Still further, while various alternative embodiments as to the various aspects, concepts and features of the inventions (such as alternative materials, structures, configurations, methods, circuits, devices and components, alternatives as to form, fit and function, and so on) may be described herein, such descriptions are not intended to be a complete or exhaustive list of available alternative embodiments, whether presently known or later developed. Those skilled in the art may readily adopt one or more of the inventive aspects, concepts or features into additional embodiments and uses within the scope of the general inventive concepts even if such embodiments are not expressly disclosed herein. Additionally, even though some features, concepts or aspects of the inventions may be described herein as being a preferred arrangement or method, such description is not intended to suggest that such feature is required or necessary unless expressly so stated. Still further, exemplary or representative values and ranges may be included to assist in understanding the present disclosure; however, such values and ranges are not to be construed in a limiting sense and are intended to be critical values or ranges only if so expressly stated. Moreover, while various aspects, features and concepts may be expressly identified herein as being inventive or forming part of an invention, such identification is not intended to be exclusive, but rather there may be inventive aspects, concepts and features that are fully described herein without being expressly identified as such or as part of a specific invention.

## Claims

1. A suction valve stem (20), comprising:
a button head (22), and
a stem body (24) connecting to the button head (22),
wherein the stem body (24) comprises:
an air passage through the center bore of the stem body (24), wherein the air passage comprises a side opening (28) disposed on side surface of the stem body (24) and a bottom opening (32) formed at a bottom end of the stem body (24),
a recessed slot (26), formed on the stem body (24) below the button head (22) and above the side opening (28), wherein the recessed slot (26) is disposed 90 degrees from the side opening (28) and is disposed longitudinally along an outer surface of the stem body (24),
a sealing area (34), disposed on the stem body (24) below the side opening (28), wherein the sealing area (34) extends from a lower edge of the side opening (28) to a bottom end of the stem body (24) such that the sealing area (34) is in fluid communication with the bottom end of the stem body (24),
a first flat (36), formed on the stem body (24), wherein the first flat (36) is formed 90 degrees from the side opening (28) and extends along the stem body (24) to the bottom end of the stem body (24) such that the first flat (36) has a fluid communication to the bottom end of the stem body (24), and
a second flat (36) formed on the stem body (24), wherein the second flat (36) is formed 90 degrees from the side opening (28) and extends along the stem body (24) to the bottom end of the stem body (24) such that the second flat (36) has a fluid communication to the bottom end of the stem body (24) and is separated from the first flat (36) by the sealing area (34).

2. The suction valve stem of claim 1, wherein the sealing area (34) extends to the bottom end of the stem body (24).

3. The suction valve stem of claim 1, wherein the sealing area (34) extends to a lower edge of the side opening (28).

4. The suction valve stem of claim 1, wherein the minimum width of the sealing area (34) is greater than the diameter of the side opening (28).

5. The suction valve stem of claim 1, wherein the maximum width of the sealing area (34) is less than the perimeter of the stem body (24).

6. The suction valve stem of claim 1, wherein the cross sectional view of the flats (36) is a plane shape.

7. The suction valve stem of claim 1, wherein the cross sectional view of the flats (36) is a concave shape.

8. The suction valve stem of claim 1, wherein the cross sectional view of the flats (36) is a slot shape.

9. The suction valve stem of claim 1, wherein the cross sectional view of the flats (36) is a pit shape.

10. The suction valve stem of claim 1, wherein the cross sectional view of the flats (36) is a four-flat shape.

11. A suction valve (10), comprising:
a suction valve stem (20) according to claim 1,
a spring (30),
a spring cup (50), and
a boot (40),
wherein the bottom end of the suction valve stem (20) extends through the spring (30), the spring cup (50) and the boot (40).

12. An endoscopic valve kit, comprising:
a suction valve (10) according to claim 11,
an air/water valve, and
a biopsy valve.

## Patentansprüche

1. Saugventilschaft (20), umfassend:
einen Flachkopf (22) und
einen Schaftkörper (24), der mit dem Flachkopf (22) verbunden ist,
wobei der Schaftkörper (24) Folgendes umfasst:
einen Luftdurchgang durch die Mittelbohrung des Schaftkörpers (24), wobei der Luftdurchgang eine Seitenöffnung (28), die an einer Seitenfläche des Schaftkörpers (24) angeordnet ist, und eine Bodenöffnung (32), die an einem unteren Ende des Schaftkörpers (24) ausgebildet ist, umfasst,
einen eingelassenen Schlitz (26), der an dem Schaftkörper (24) unterhalb des Flachkopfs (22) und oberhalb der Seitenöffnung (28) ausgebildet ist, wobei der eingelassene Schlitz (26) in einem Winkel von 90 Grad von der Seitenöffnung (28) und in Längsrichtung entlang einer Außenfläche des Schaftkörpers (24) angeordnet ist,
einen Dichtbereich (34), der an dem Schaftkörper (24) unterhalb der Seitenöffnung (28) angeordnet ist, wobei sich der Dichtbereich (34) von einem unteren Rand der Seitenöffnung (28) zu einem unteren Ende des Schaftkörpers (24) derart erstreckt, dass der Dichtbereich (34) in Fluidverbindung mit dem unteren Ende des Schaftkörpers (24) steht,
eine erste Abflachung (36), die an dem Schaftkörper (24) ausgebildet ist, wobei die erste Abflachung (36) in einem Winkel von 90 Grad von der Seitenöffnung (28) ausgebildet ist und sich entlang des Schaftkörpers (24) zu dem unteren Ende des Schaftkörpers (24) derart erstreckt, dass die erste Abflachung (36) eine Fluidverbindung zu dem unteren Ende des Schaftkörpers (24) aufweist, und
eine zweite Abflachung (36), die an dem Schaftkörper (24) ausgebildet ist, wobei die zweite Abflachung (36) in einem Winkel von 90 Grad von der Seitenöffnung (28) ausgebildet ist und sich entlang des Schaftkörpers (24) zu dem unteren Ende des Schaftkörpers (24) derart erstreckt, dass die zweite Abflachung (36) eine Fluidverbindung zu dem unteren Ende des Schaftkörpers (24) aufweist und durch den Dichtbereich (34) von der ersten Abflachung (36) getrennt ist.

2. Saugventilschaft nach Anspruch 1, wobei sich der Dichtbereich (34) zu dem unteren Ende des Schaftkörpers (24) erstreckt.

3. Saugventilschaft nach Anspruch 1, wobei sich der Dichtbereich (34) zu einem unteren Rand der Seitenöffnung (28) erstreckt.

4. Saugventilschaft nach Anspruch 1, wobei die Mindestbreite des Dichtbereichs (34) größer ist als der Durchmesser der Seitenöffnung (28).

5. Saugventilschaft nach Anspruch 1, wobei die Maximalbreite des Dichtbereichs (34) kleiner ist als der Umfang des Schaftkörpers (24) .

6. Saugventilschaft nach Anspruch 1, wobei die Querschnittsansicht der Abflachungen (36) eine ebene Form ist.

7. Saugventilschaft nach Anspruch 1, wobei die Querschnittsansicht der Abflachungen (36) eine konkave Form ist.

8. Saugventilschaft nach Anspruch 1, wobei die Querschnittsansicht der Abflachungen (36) eine Schlitzform ist.

9. Saugventilschaft nach Anspruch 1, wobei die Querschnittsansicht der Abflachungen (36) eine Vertiefungsform ist.

10. Saugventilschaft nach Anspruch 1, wobei die Querschnittsansicht der Abflachungen (36) eine Form mit vier Abflachungen ist.

11. Saugventil (10), umfassend:
einen Saugventilschaft (20) nach Anspruch 1,
eine Feder (30),
einen Federteller (50) und
eine Schutzkappe (40),
wobei sich das untere Ende des Saugventilschafts (20) durch die Feder (30), den Federteller (50) und die Schutzkappe (40) erstreckt.

12. Endoskopisches Ventilset, umfassend:
ein Saugventil (10) nach Anspruch 11,
ein Luft-/Wasserventil und
ein Biopsieventil.

## Revendications

1. Tige de soupape d'aspiration (20), comprenant :
une tête de bouton (22), et
un corps de tige (24) relié à la tête de bouton (22),
dans laquelle le corps de tige (24) comprend :
un passage d'air à travers l'alésage central du corps de tige (24), dans laquelle le passage d'air comprend une ouverture latérale (28) disposée sur la surface latérale du corps de tige (24) et une ouverture inférieure (32) formée à une extrémité inférieure du corps de tige (24),
une fente encastrée (26), formée sur le corps de tige (24) sous la tête de bouton (22) et au-dessus de l'ouverture latérale (28), dans laquelle la fente encastrée (26) est disposée à 90 degrés de l'ouverture latérale (28) et est disposée longitudinalement le long d'une surface extérieure du corps de tige (24),
une zone d'étanchéité (34), disposée sur le corps de tige (24) sous l'ouverture latérale (28), dans laquelle la zone d'étanchéité (34) se prolonge d'un bord inférieur de l'ouverture latérale (28) à une extrémité inférieure du corps de tige (24) de sorte que la zone d'étanchéité (34) soit en communication fluidique avec l'extrémité inférieure du corps de tige (24),
un premier méplat (36), formé sur le corps de tige (24), dans laquelle le premier méplat (36) est formé à 90 degrés de l'ouverture latérale (28) et se prolonge le long du corps de tige (24) jusqu'à l'extrémité inférieure du corps de tige (24) de sorte que le premier méplat (36) ait une communication fluidique avec l'extrémité inférieure du corps de tige (24), et
un second méplat (36) formé sur le corps de tige (24), dans laquelle le second méplat (36) est formé à 90 degrés de l'ouverture latérale (28) et se prolonge le long du corps de tige (24) jusqu'à l'extrémité inférieure du corps de tige (24) de sorte que le second méplat (36) ait une communication fluidique avec l'extrémité inférieure du corps de tige (24) et soit séparé du premier méplat (36) par la zone d'étanchéité (34) .

2. Tige de soupape d'aspiration selon la revendication 1, dans laquelle la zone d'étanchéité (34) se prolonge jusqu'à l'extrémité inférieure du corps de tige (24).

3. Tige de soupape d'aspiration selon la revendication 1, dans laquelle la zone d'étanchéité (34) se prolonge jusqu'à un bord inférieur de l'ouverture latérale (28).

4. Tige de soupape d'aspiration selon la revendication 1, dans laquelle la largeur minimale de la zone d'étanchéité (34) est supérieure au diamètre de l'ouverture latérale (28).

5. Tige de soupape d'aspiration selon la revendication 1, dans laquelle la largeur maximale de la zone d'étanchéité (34) est inférieure au périmètre du corps de tige (24).

6. Tige de soupape d'aspiration selon la revendication 1, dans laquelle la vue en coupe des méplats (36) est une forme plane.

7. Tige de soupape d'aspiration selon la revendication 1, dans laquelle la vue en coupe des méplats (36) est une forme concave.

8. Tige de soupape d'aspiration selon la revendication 1, dans laquelle la vue en coupe des méplats (36) est une forme de fente.

9. Tige de soupape d'aspiration selon la revendication 1, dans laquelle la vue en coupe des méplats (36) est une forme de fosse.

10. Tige de soupape d'aspiration selon la revendication 1, dans laquelle la vue en coupe des méplats (36) est une forme à quatre méplats.

11. Soupape d'aspiration (10), comprenant :
une tige de soupape d'aspiration (20) selon la revendication 1,
un ressort (30),
une cuvette de ressort (50), et
un soufflet (40),
dans laquelle l'extrémité inférieure de la tige de soupape d'aspiration (20) se prolonge à travers le ressort (30), la cuvette de ressort (50) et le soufflet (40).

12. Kit de soupape endoscopique, comprenant :
une soupape d'aspiration (10) selon la revendication 11,
une soupape air/eau, et
une soupape de biopsie.
